# EUROPÄISCHE PATENTSCHRIFT

(11) **EP 3 253 758 B1**
(45) Veröffentlichungstag und Bekanntmachung des Hinweises auf die Patenterteilung: **03.04.2019**
(21) Anmeldenummer: 16702153.4
(22) Anmeldetag: 02.02.2016
(51) Int. Cl.: C07D 487/04, A61K 31/4985, A61P 9/00, A61P 9/10, A61P 9/12, A61P 25/28, A61P 15/10

(54) **N-SUBSTITUIERTE 8-[(2,6-DIFLUOROBENZYL)OXY]-2,6-DIMETHYLIMIDAZO[1,2-A]PYRAZIN-3-CARBOXAMID-DERIVATE ALS STIMULATOREN DER LÖSLICHEN GUANYLATCYCLASE (SGC) ZUR BEHANDLUNG VON KARDIOVASKULÄREN ERKRANKUNGEN**
N-SUBSTITUTED 8-[(2,6-DIFLUOROBENZYL)OXY]-2,6-DIMETHYLIMIDAZO[1,2-A]PYRAZINE-3-CARBOXAMIDE-DERIVATIVES AS STIMULATORS OF THE SOLUBLE GUANYLATCYCLASE (SGC) FOR THE TREATMENT OF CARDIOVASCULAR DISEASES
DÉRIVÉS SUBSTITUÉS-N DE CARBOXAMIDE 8-[(2,6-DIFLUOROBENZYL)OXY]-2,6-DIMETHYLIMIDAZO[1,2-A]PYRAZINE-3- EN TANT QUE STIMULATEURS DE LA GUANYLATCYCLASE SOLUBLE (GCS) POUR LE TRAITEMENT DE MALADIES CARDIOVASCULAIRES

(30) Priorität: 05.02.2015 EP 15153959
(43) Veröffentlichungstag der Anmeldung: 13.12.2017
(73) Patentinhaber: Bayer Pharma Aktiengesellschaft, 13353 Berlin (DE)
(72) Erfinder: VAKALOPOULOS, Alexandros, 40721 Hilden (DE); BROCKSCHNIEDER, Damian, 42781 Haan (DE); WUNDER, Frank, 42117 Wuppertal (DE); STASCH, Johannes-Peter, 00046 Grottarerrata (RM) (IT); MARQUARDT, Tobias, 42115 Wuppertal (DE); DIETZ, Lisa, 42111 Wuppertal (DE); LI, Volkhart Min-Jian, 42553 Velbert (DE)
(74) Vertreter: BIP Patents
(86) Internationale Anmeldenummer: PCT/EP2016/052123
(87) Internationale Veröffentlichungsnummer: WO 2016/124564

(56) Entgegenhaltungen:
- WO-A1-2014/068099
- WO-A1-2015/018808

## Beschreibung

Die vorliegende Anmeldung betrifft neue substituierte Imidazo[1,2-a]pyrazincarboxamide, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in WO 89/03833-A1 und WO 96/34866-A1 sind verschiedene Imidazo[1,2-a]pyrazin-Derivate beschrieben, die zur Behandlung von Erkrankungen verwendet werden können. Die WO 2014/068099 A1 betrifft Verbindungen, die sich von den Verbindungen der vorliegenden Erfindung in der Grundstruktur (Imidazopyridin statt Imidazopyrazin) und in der Definition des Substituenten R¹ unterscheiden. Die WO 2015/018808 A1 betrifft Verbindungen, die sich von den Verbindungen der vorliegenden Erfindung in der Definition des Substituenten R¹ unterscheiden.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten, ihrer Löslichkeit und/oder ihres Metabolismus-Profils und/oder ihrer Dosis-Wirkungsbeziehung.

Die vorliegende Erfindung betrifft Verbindungen der allgemeinen Formel (I) 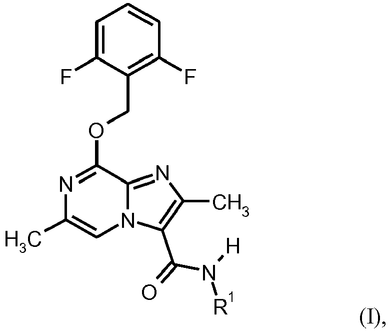 in welcher
R¹ für eine Gruppe der Formel 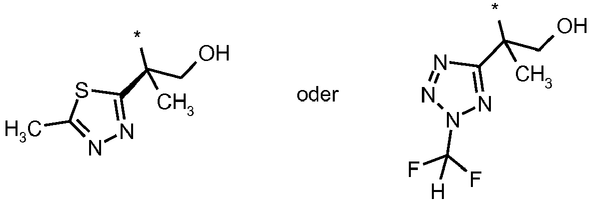 oder 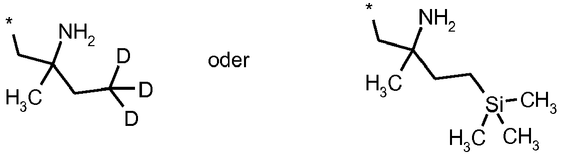 oder 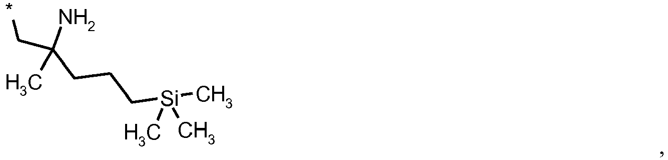 steht, wobei
* für die Anknüpfstelle an das Stickstoffatom steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Im Rahmen der vorliegenden Erfindung haben die Substituenten, soweit nicht anders spezifiziert, die folgende Bedeutung:
In den Formeln der Gruppe, für die R¹ stehen kann, steht der Endpunkt der Linie, an dem ein Zeichen * steht, nicht für ein Kohlenstoffatom beziehungsweise eine CH₂-Gruppe, sondern ist Bestandteil der Bindung zu dem jeweils bezeichneten Atom, an das R¹ gebunden sind.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der Formel (I), in welcher
R¹ für eine Gruppe der Formel 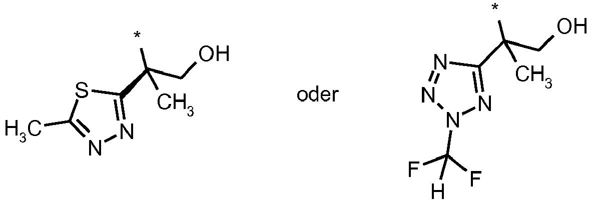 oder 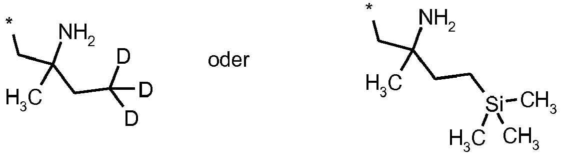 oder, steht, wobei
* für die Anknüpfstelle an das Stickstoffatom steht,
sowie ihre *N*-Oxide, Salze, Solvate, Salze der *N*-Oxide und Solvate der *N*-Oxide und Salze.

Bevorzugt ist im Rahmen der vorliegenden Erfindung dieVerbindung mit dem systematischen Namen *ent-*8-[(2,6-Difluorbenzyl)oxy]-N-[(2*S*)-1-hydroxy-2-(5-methyl-1,3,4-thiadiazol-2-yl)propan-2-yl]-2,6-dimethylimidazo[1,2-a]pyrazin-3-carboxamid und der Strukturformel 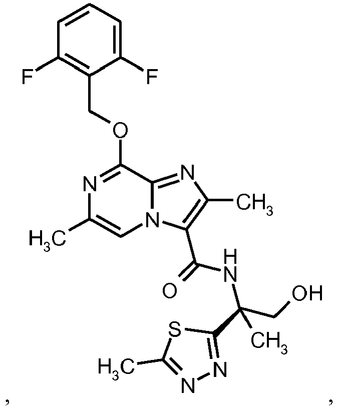 sowie ihre Salze, ihrer Solvate oder der Solvate ihrer Salze.

Bevorzugt ist im Rahmen der vorliegenden Erfindung dieVerbindung mit dem systematischen Namen *rac-*8-[(2,6-Difluorbenzyl)oxy]-N-{2-[2-(difluormethyl)-2H-tetrazol-5-yl]-1-hydroxypropan-2-yl}-2,6-dimethylimidazo[1,2-a]pyrazin-3-carboxamid und der Strukturformel 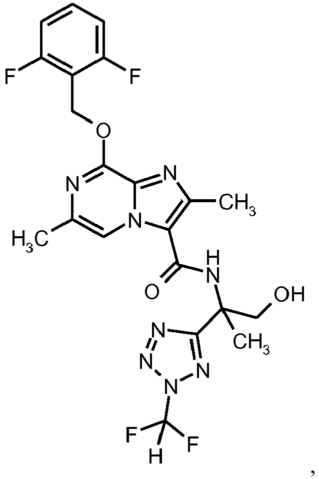 sowie ihre Salze, ihrer Solvate oder der Solvate ihrer Salze.

Bevorzugt ist im Rahmen der vorliegenden Erfindung dieVerbindung mit dem systematischen Namen *ent-*N-[2-Amino-2-methyl(4,4,4-²H₃)butyl]-8-[(2,6-difluorbenzyl)oxy]-2,6-dimethylimidazo[1,2-a]pyrazin-3-carboxamid (Enantiomer A) und der Strukturformel 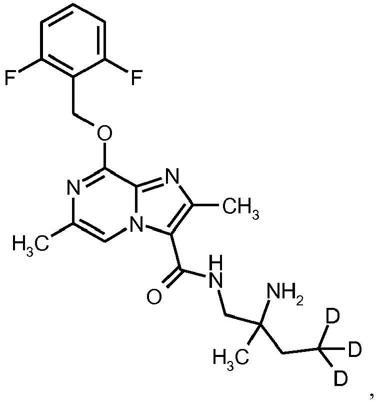 sowie ihre Salze, ihrer Solvate oder der Solvate ihrer Salze.

Bevorzugt ist im Rahmen der vorliegenden Erfindung die Verbindung mit dem systematischen Namen *ent-*N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer A) und der Strukturformel 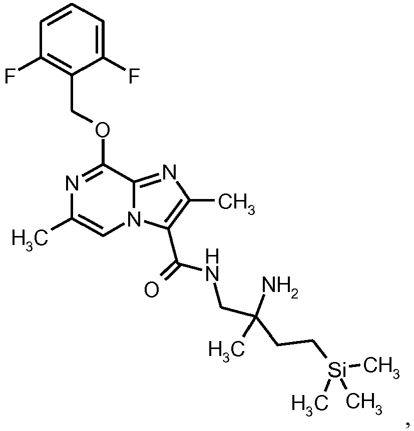 sowie ihre Salze, ihrer Solvate oder der Solvate ihrer Salze.

Bevorzugt ist im Rahmen der vorliegenden Erfindung dieVerbindung mit dem systematischen Namen *ent-*N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer B) und der Strukturformel 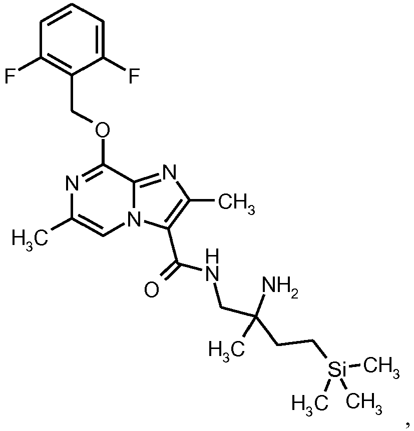 sowie ihre Salze, ihrer Solvate oder der Solvate ihrer Salze.

Die als bevorzugt genannten Restedefinitionen gelten sowohl für die Verbindungen der Formel (I) als auch in entsprechender Weise für alle Zwischenprodukte.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 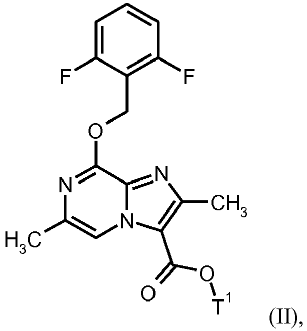 in welcher
- T¹: für (C₁-C₄)-Alkyl oder Benzyl steht,
in einem inerten Lösungsmittel in Gegenwart einer geeigneten Base oder Säure zu einer Carbonsäure der Formel (III) 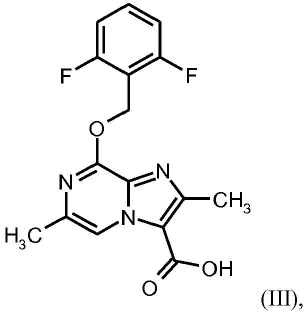 umsetzt und diese in der Folge in einem inerten Lösungsmittel unter Amidkupplungsbedingungen mit einem Amin der Formel (IV-A), (IV-B), (IV-C) oder (IV-D) 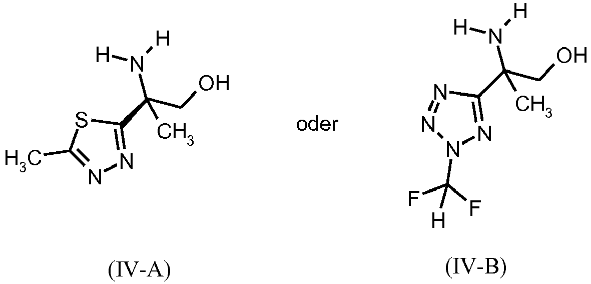 oder 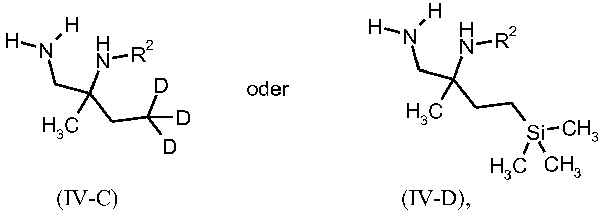 in welchem R² für eine Amino-Schutzgruppe, wie beispielsweise tert.-Butoxycarbonyl, Benzyloxycarbonyl oder Benzyl steht, umsetzt,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Das beschriebene Herstellverfahren kann durch das folgende Syntheseschema (Schema 1) beispielhaft verdeutlicht werden: 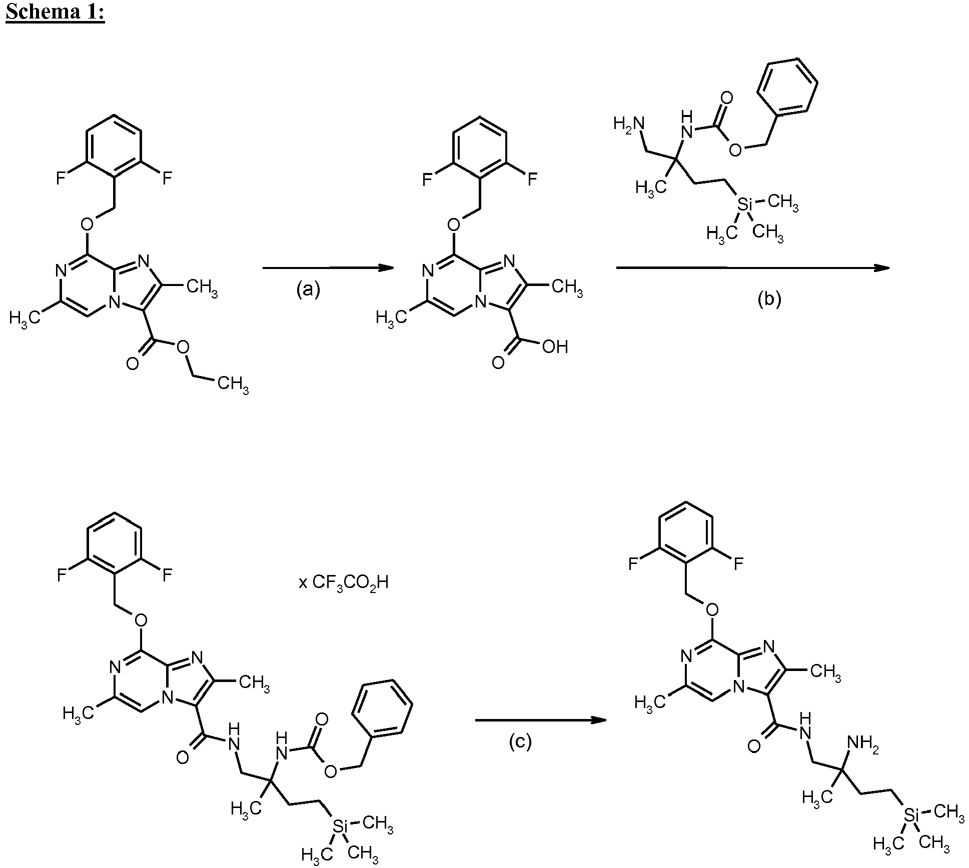
[(a) Natriumhydroxid, 1,4-Dioxan, 90°C; (b) HATU, N,N-Diisopropylethylamin, DMF, Raumtemperatur; (c)Wasserstoff, 10%iges Palladium auf Aktivkohle, TFA, Ethanol].

Die Verbindungen der Formeln (IV-A), (IV-B), (IV-C) und (IV-D) sind kommerziell erhältlich, literaturbekannt oder können in Analogie zu literaturbekannten Verfahren hergestellt werden.

Inerte Lösungsmittel für die Verfahrensschritte (III) + (IV) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N*,*N*-Dimethylformamid, *N*,*N*-Dimethylacetamid, *N*,*N'*-Dimethylpropylenharnstoff (DMPU) oder *N-*Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (III) + (IV) → (I) und eignen sich beispielsweise Carbodiimide wie *N*,*N'*-Diethyl-, *N*,*N'*-Dipropyl-, *N*,*N'*-Diisopropyl-, *N*,*N'*-Dicyclohexylcarbodiimid (DCC) oder *N*-(3-Dimethylaminopropyl)-*N'*-ethylcarbodiimid-Hydrochlorid (EDC), Phosgen-Derivate wie *N*,*N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N*,*N*,2-trimethylpropl-en-1-amin, Cyanophosphonsäurediethylester, Bis-(2-oxo-3 -oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (Py-BOP), *O*-(Benzotriazol-1-yl)-*N*,*N*,*N',N'*-tetramethyluronium-tetrafluorborat (TBTU), *O*-(Benzotriazol-1-yl)-*N*,*N*,*N'*,*N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N*,*N*,*N'*,*N'*-tetramethyluronium-hexafluorphosphat (HATU) oder *O*-(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N*,*N*-Diisopropylethylamin. Bevorzugt wird TBTU in Verbindung mit N-Methylmorpholin, HATU in Verbindung mit *N*,*N*-Diisopropylethylamin oder 1-Chlor-*N*,*N*,2-trimethylprop-1-en-1amin verwendet.

Die Kondensationen (III) + (IV) → (I) und wird im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (III) auch zunächst in das entsprechende Carbonsäurechlorid überführt werden und dieses dann direkt oder in einer separaten Umsetzung mit einem Amin der Formel (IV) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, Sulfurylchlorid oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Als Amino- Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert*.-Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch durch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionschritten vorgenommen werden.

Die Verbindungen der Formel (II) sind literaturbekannt oder können hergestellt werden, indem eine Verbindung der Formel (V) 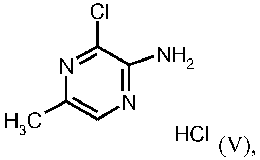 in einem inerten Lösungsmittel in Gegenwart einer geeigneten Base mit einer Verbindung der Formel (VI) 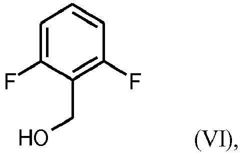 zu einer Verbindung der Formel (VII) 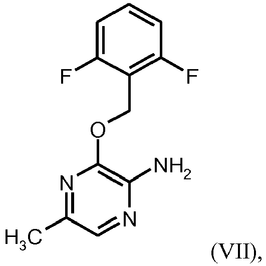 umgesetzt wird, und diese anschliessend in einem inerten Lösungsmittel mit einer Verbindung der Formel (VIII) 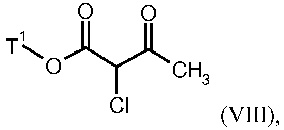 in welcher T¹ jeweils die oben angegebenen Bedeutung hat,
umgesetzt wird.

Das beschriebene Verfahren wird durch das nachfolgende Schema (Schema 2) beispielhaft verdeutlicht: 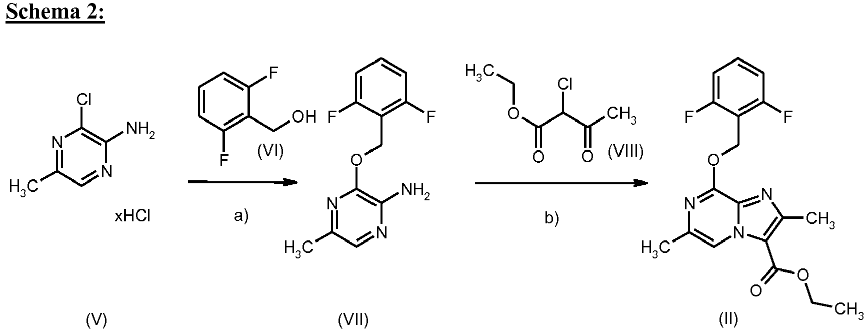
[(a) Kalium-tert-butylat, 1,2-Dimethoxyethan, 80°C; (b) Ethanol, Molekularsieb, Rückfluss].

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 3 gezeigt. 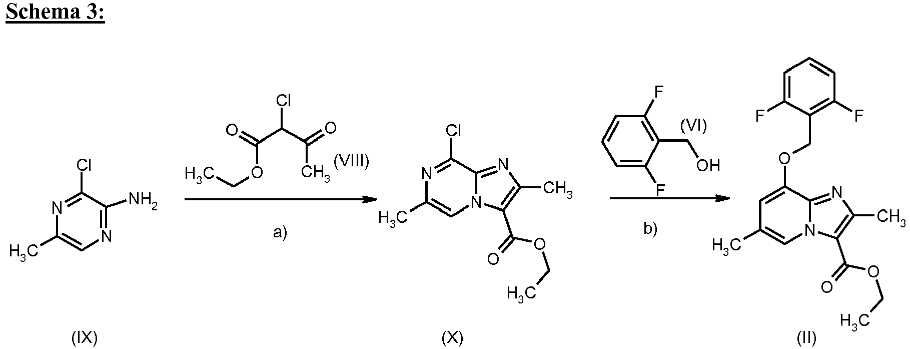
[a): EtOH, Molekularsieb, Rückfluss; b): Kalium-tert-butylat, 1,2-Dimethoxyethan, 80°C].

Inerte Lösungsmittel für den Verfahrensschritt (V) + (VI) → (VII) bzw. (X) + (VI) → (II) sind beispielsweise Ether wie Diethylether, Dioxan, Tetrahydrofuran, Dimethoxymethan, Glykoldimethylether oder Diethylenglykoldimethylether oder andere Lösungsmittel wie Aceton, Methylethylketon, Essigsäureethylester, Acetonitril, *N*,*N*-Dimethylformamid, *N*,*N*-Dimethylacetamid, Dimethylsulfoxid, *N,N'-*Dimethylpropylenharnstoff (DMPU), *N*-Methylpyrrolidon (NMP). Ebenso ist es möglich, Gemische der genannten Lösungsmittel einzusetzen. Bevorzugt wird Dimethoxyethan verwendet.

Als Basen für den Verfahrensschritt (V) + (VI) → (VII) bzw. (X) + (VI) → (II) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N*,*N*-Diisopropylethylamin, Pyridin, 4-(*N*,*N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®). Bevorzugt wird Natrium- oder Kalium-tert.-butylat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyrazin-Grundgerüst (VII) + (VIII) → (II) bzw. (VIII) + (IX) → (X) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (VII) + (VIII) → (II) bzw. (VIII) + (IX) → (X) erfolgt optional in Gegenwart wasserziehender Reaktionszusätze, beispielsweise in Gegenwart von Molekularsieb (3Å oder 4Å Porengröße) oder mittels Wasserabscheider. Die Umsetzung (VII) + (VIII) → (II) bzw. (VIII) + (IX) → (X) erfolgt unter Verwendung eines Überschusses des Reagenzes der Formel (VIII), beispielsweise mit 1 bis 20 Äquivalenten des Reagenzes (VIII), gegebenenfalls unter Zusatz von Basen (wie z.B. Natriumhydrogencarbonat) wobei die Zugabe dieses Reagenzes einmalig oder in mehreren Portionen erfolgen kann.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen wirken als potente Stimulatoren der löslichen Guanylatcyclase, besitzen wertvolle pharmakologische Eigenschaften, und weist ein verbessertes therapeutisches Profil auf, wie beispielsweise hinsichtlich ihrer *in-vivo* Eigenschaften und/oder ihres pharmakokinetischen Verhaltens und/oder metabolischen Profils. Sie eignet sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärkt die erfindungsgemäße Verbindung die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus kann die erfindungsgemäße Verbindung auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronischobstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weitere Offenbarung der vorliegenden Erfindung ist die Verwendung der erfindungsgemäßen Verbindungen zur Behandlung und/oder Prophylaxe von Erkrankungen, insbesondere der zuvor genannten Erkrankungen.

Weitere Offenbarung der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.
Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.
Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.
Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.
Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), Edoxaban (DU-176b), Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan, Embursartan, Irbesartan, Olmesartan, Eprosartan oder Azilsartan oder einem dualen Angiotensin AII-Antagonisten/NEP-Inhibitor, wie beispielsweise und vorzugsweise LCZ696 (Valsartan/Sacubitril), verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut (= getrocknet)
- aq.: wässrige Lösung
- ber.: berechnet
- Boc: *tert*-Butyloxycarbonyl
- br.: Verbreitertes Signal (NMR Kupplungsmuster)
- CAS-Nr.: Chemical Abstracts Service Nummer
- Cbz: Benzyloxycarbonyl
- δ: Verschiebung im NMR Spektrum (Angabe in ppm)
- d: Dublett (NMR-Kupplungsmuster)
- DC: Dünnschichtchromatographie
- DCI: direkte chemische Ionisation (bei MS)
- DMAP: 4-*N*,*N*-Dimethylaminopyridin
- DMF: Dimethylformamid
- DMSO: Dimethylsulfoxid
- EDCI: *N*-[3-(Dimethylamino)propyl]-N'-ethylcarbodiimid
- d. Th.: der Theorie (bei Ausbeute)
- *ent*: enantiomerenrein
- eq.: Äquivalent(e)
- ESI: Elektrospray-Ionisation (bei MS)
- Et: Ethyl
- gef.: gefunden
- h: Stunde(n)
- HATU: N-[(Dimethylamino)(3H-[1,2,3]triazolo[4,5-b]-pyridin-3-yloxy)methylen]-N-methylmethanaminiumhexafluorophosphat
- HOBT: 1H-Benzotriazol-1-ol
- HPLC: Hochdruck-, Hochleistungsflüssigchromatographie
- HRMS: hochaufgelöste Massenspektrometrie
- ID: Innendurchmesser
- konz.: konzentriert
- LC-MS: Flüssigchromatographie-gekoppelte Massenspektrometrie
- LiHMDS: Lithiumhexamethyldisilazid
- m: Multiplett
- Me: Methyl
- min: Minute(n)
- MS: Massenspektrometrie
- NMR: Kernresonanzspektrometrie
- PDA: Photodiodenarraydetektor
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- *rac*: racemisch
- *rel*: relative Stereochemie
- R_{F}: Retentionsfaktor (bei Dünnschichtchromatographie)
- RT: Raumtemperatur
- Rₜ: Retentionszeit (bei HPLC)
- s: Singulett (NMR Kupplungsmuster)
- t: Triplett (NMR Kupplungsmuster)
- THF: Tetrahydrofuran
- TBTU: (Benzotriazol-1-yloxy)bisdimethylaminomethyliumfluoroborat
- UPLC-MS: Ultradruck-Flüssigchromatographiegekoppelte Massenspektrometrie
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

### LC-MS- und HPLC-Methoden:

### Methode 1 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm

### Methode 2 (LC-MS):

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 210 - 400 nm.

### Methode 3 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 4 (präparative HPLC):

Chromatorex C18 10µ 250x20 mm Gradient: A= Wasser + 0,5% Ameisensäure, B = Acetonitril, 0min = 5% B, 3min = 5% B vorspülen ohne Substanz, dann Injektion, 5 min = 5% B, 25min = 30% B, 38min = 30% B, 38.1 min = 95% B, 43.00 min = 95% B, 43.01 min= 5% B, 48.0min = 5% B; Flussrate 20 ml/min, Wellenlänge 210 nm.

### Methode 5 (präparative HPLC):

Chromatorex C18 10µ 250x20 mm Gradient: A= Wasser + 0.5% Ameisensäure, B = Acetonitril, 0 min = 5% B, 3min = 5% B vorspülen ohne Substanz, dann Injektion, 5 min = 5% B, 25 min = 50% B, 38 min = 50% B, 38.1 min = 95% B, 43.00 min = 95% B, 43.01 min = 5% B, 48.0 min = 5% B; Flussrate 20 ml/min, Wellenlänge 210 nm.

### Methode 6 (präparative HPLC):

XBridge Prep. C18 5µ 50x19 mm Gradient: A= Wasser + 0.5% Ammoniumhydroxid, B= Acetonitril, 0min = 5% B, 3 min = 5% B vorspülen ohne Substanz, dann Injektion, 5 min = 5% B, 25 min = 50% B, 38.0 min = 50% B, 38.1 min = 95% B, 43.00 min = 95% B, 43.01 min= 5% B, 48.0 min= 5% B; Flussrate 15 ml/min, Wellenlänge 210 nm

### Methode 7 (LC-MS):

Instrument MS: Waters (Micromass) QM; Instrument HPLC: Agilent 1100 Serie; Säule : Agient ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A → 4.5 min 5% A ; Ofen: 40°C; Fluss: 1.75 ml/min; UV-Detektion: 210 nm.

### Methode 8 (LC-MS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 9 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, mit Gradient; Fluss: 40 ml/min; UV-Detektion: DAD; 210 - 400 nm).
bzw.:
Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäure, Eluent B: Acetonitril (ULC) + 0.05% Ameisensäure, mit Gradient; Fluss: 40 ml/min; UV-Detektion: DAD; 210 - 400 nm).

### Methode 10 (LC-MS):

Instrument MS: Waters SQD; Instrument HPLC: Waters UPLC; Saeule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 11 (MS):

Instrument: Waters ZQ 2000; Elektrospray-Ionisierung; Eluent A: 1 l Wasser + 0.25 ml 99%ige Ameisensäure, Eluent B: 1 l Acetonitril + 0.25 ml 99%ige Ameisensäure; 25% A, 75% B; Fluss: 0.25 ml/min.

### Methode 12 (DCI-MS):

Instrument: Thermo Fisher-Scientific DSQ; chemische Ionisierung; Reaktantgas NH₃; Quellentemperatur: 200°C; Ionisierungsenergie 70eV.

### Methode 13 (LC-MS):

Instrument MS: Waters (Micromass) Quattro Micro; Instrument HPLC: Agilent 1100 Serie; Säule: YMC-Triart C18 3 µ 50 x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm.

### Methode 14 (GC-MS):

Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 15 (LC-MS, analytisch):

Instrument: Agilent MS Quad 6150; HPLC: Agilent 1290; Säule: Waters Acquity UPLC HSS T3 1.8 µ 50 x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1.20 ml/min; UV-Detektion: 205 - 305 nm.

### Methode 16 (LC-MS, analytisch):

Instrument MS: Waters (Micromass) Quattro Micro; Instrument Waters UPLC Acquity; Säule : Waters BEH C18 1.7 µ 50 x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A → 2.51 min 10% A → 3.0 min 10% A; Ofen: 40°C; Fluss: 0.5 ml/min; UV-Detektion: 210 nm.

### Methode 17 (LC-MS):

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

### Weitere Angaben:

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. saure Funktionalitäten enthalten. Ein solches Salz kann durch verschiedene dem Fachmann bekannte Methoden in die entsprechende freie Base bzw. Säure überführt werden.

So können die Trifluoracetat-, Formiat- oder Ammonium-Salze durch Ausschütteln einer organischen Lösung oder Suspension mit gesättigter, wässriger Natriumhydrogencarbonatlösung in die salzfreie Form überführt werden.

Des Weiteren können Amidine als freie Verbindungen oder anteilig (abhängig von der Präparation bei Beteiligung von Essigsäure) als Acetat-Salze oder Acetat-Solvate vorliegen.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Weiterhin können die erfindungsgemäßen sekundären Amide als Rotationsisomere/ Isomerengemische, insbesondere bei NMR-Untersuchungen, vorliegen. Reinheitsangaben beziehen sich in der Regel auf entsprechende Peak-Integrationen im LC/MS-Chromatogramm, können aber zusätzlich auch unter Zuhilfenahme des ¹H-NMR-Spektrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Alle Angaben in ¹H-NMR-Spektren geben die Chemischen Verschiebungen δ in ppm an.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Die Methyl-Gruppe des chemischen Systems "2-Methylimidazo[1,2-a]pyrazin" erscheint in ¹H-NMR-Spektren als Singulett (oftmals in DMSO-d₆ und im Bereich von 2.40 - 2.60 ppm) und ist etweder klar als solches erkennbar, ist mit den Lösungsmittelsignalen überlagert oder liegt vollständig unter den Signalen der Lösungsmittel.

Schmelzpunkte und Schmelzbereiche, soweit angegeben, sind nicht korrigiert.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### 3-[(2,6-Difluorbenzyl)oxy]-5-methylpyrazin-2-amin

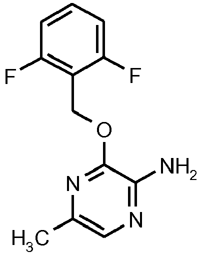

Zu einer Lösung von 2.71 g (2,6-Difluorphenyl)methanol [CAS-Nr.: 19064-18-7] (18.8 mmol, 1.3 eq.) in 120 ml 1,2-Dimethoxyethan wurden 4.86 g Kalium-tert-butylat (43.3 mmol, 3.0 eq.) gegeben und das Gemisch wurde 60 min bei RT gerührt. Anschließend wurden 2.60 g 2-Amino-3-chlor-5-methylpyrazin Hydrochlorid [CAS-Nr.: 89182-14-9] (14.4 mmol, 1.0 eq.) zugegeben und die Mischung wurde über Nacht bei 80°C gerührt. Nach Abkühlen auf Raumtemperatur wurde gesättigte wässrige Natriumhydrogencarbonatlösung zugegeben und die wässrige Phase dreimal mit Dichlormethan extrahiert. Die vereinigten organischen Phasen wurden mit gesättigter wässriger Natriumchloridlösung gewaschen, mit Magnesiumsulfat getrocknet, filtriert und eingeengt. Der Rückstand wurde mittels Biotage Isolera (340 g Kieselgelkartusche, Cyclohexan /Essigsäureethylester Gradient, 10% -> 72% Essigsäureethylester) gereingt. Es wurden 1.77 g der Titelverbindung (39% d. Th., Reinheit 85%) erhalten.
LC-MS (Methode 2): Rₜ = 0.94 min
MS (ESpos): m/z = 252 (M+H)⁺
¹H-NMR (400 MHz, DMSO-d₆): δ [ppm] = 2.20 (s, 3H), 5.35 (s, 2H), 5.88 (s, 2H), 7.09 - 7.23 (m, 2H), 7.37 (s, 1H), 7.46 - 7.57 (m, 1H).

### Beispiel 2A

### Ethyl-8-[(2,6-difluorbenzyl)oxy]-2,6-dimethylimidazo[1,2-a]pyrazin-3-carboxylat

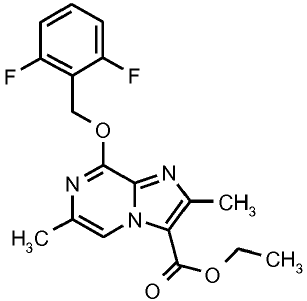

Eine Lösung von 1.77 g 3-[(2,6-Difluorbenzyl)oxy]-5-methylpyrazin-2-amin (7.05 mmol, 1.0 eq.) aus Beispiel 1A in 50 ml Ethanol wurde mit Molekularsieb 4A und 11.1 g Ethyl-2-chloroacetoacetat [CAS-Nr.: 609-15-4] (70.5 mmol, 10 eq.) versetzt und über Nacht auf Rückfluss erhitzt. Anschließend wurden 11.1 g Ethyl-2-chloroacetoacetat (70.5 mmol, 10.0 eq.) zugegeben und es wurde über Nacht auf Rückfluss erhitzt. Dann wurde abfiltriert, das Filtrat eingeengt, der erhaltene Rückstand mit Diethylether ausgerührt, abfiltriert und das Filtrat eingeengt. Der Rückstand wurde zweimal mittels Biotage Isolera (120 g Kieselgelkartusche, Cyclohexan/Essigsäureethylester Gradient) gereinigt. Es wurden 0.81 g der Titelverbindung (16% d. Th., Reinheit 52%) isoliert.
LC-MS (Methode 2): Rₜ = 1.28 min
MS (ESpos): m/z = 362 (M+H)⁺

### Beispiel 3A

### 8-[(2,6-Difluorbenzyl)oxy]-2,6-dimethylimidazo[1,2-a]pyrazin-3-carbonsäure

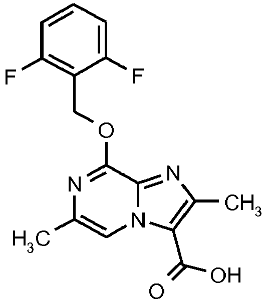

Eine Lösung von 800 mg Ethyl-8-[(2,6-difluorbenzyl)oxy]-2,6-dimethylimidazo[1,2-a]pyrazin-3-carboxylat (Reinheit 52%, 1.15 mmol, 1.0 eq.) aus Beispiel 2A in 10 ml Dioxan wurde mit 5.8 ml 1 N wässriger Natronlauge (5.8 mmol, 5 eq.) versetzt und 2 h bei RT gerührt. Anschließend wurde das Gemisch eingeengt, der Rückstand in Wasser aufgenommen und der unlösliche Feststoff wurde abfiltriert. Das Filtrat wurde mit 1 N wässriger Salzsäure angesäuert, der gebildete Feststoff wurde abfiltriert und getrocknet. Es wurden 354 mg der Titelverbindung (83% d. Th., Reinheit 90%) isoliert.

LC-MS (Methode 2): Rₜ = 0.99 min
MS (ESpos): m/z = 334 (M+H)⁺
¹H-NMR (400 MHz, DMSO-d₆): δ [ppm] = 2.41 (s, 3H), 2.54 (s, 3H verborgen unter Lösemittelsignal), 5.55 (s, 2H), 7.12 - 7.28 (m, 2H), 7.49 - 7.64 (m, 1H), 8.64 (s, 1H), 13.20 - 13.66 (br s, 1H).

### Beispiel 4A

### rac-2-Amino-2-methyl(4,4,4-²H₃)butannitril

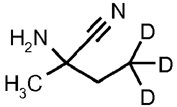

2.0 g (26.62 mmol) (4,4,4-²H₃)Butan-2-on [CAS Registry Nummer: 53389-26-7] wurden in 22.3 ml 2 N Ammoniak in Methanol vorgelegt und bei Raumtemperatur mit 1.72 g (35.14 mmol) Natriumcyanid sowie 1.88 g (35.14 mmol) Ammoniumchlorid versetzt und 4 Stunden unter Rückfluss gerührt. Das Reaktionsgemisch wurde abgekühlt, mit 40 ml Diethylether versetzt und der enthaltene Feststoff wurde abfiltriert. Das Lösungsmittel aus dem Filtrat wurde unter Normaldruck abdestilliert. Es wurden 2.75 g der Titelverbindung (51 % d. Th. bei einer Reinheit von ca. 50%) als Rückstand erhalten, der ohne weitere Reinigung in die Folgestufe eingesetzt wurde.
GC-MS (Methode 14): Rₜ = 1.66 min
MS (ESpos): m/z = 86 (M-CH₃)⁺

### Beispiel 5A

### rac-Benzyl-[2-cyan(4,4,4-²H₃)butan-2-yl]carbamat

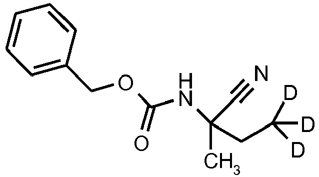

2.75 g (13.59 mmol bei einer Reinheit von ca. 50%) *rac*-2-Amino-2-methyl(4,4,4-²H₃)butannitril aus Beispiel 4A wurden in 33 ml Tetrahydrofuran/Wasser = 9/1 vorgelegt und mit 5.82 g (42.13 mmol) Kaliumcarbonat versetzt. Bei 0°C wurden langsam 2.32 g (13.59 mmol) Chlorameisensäurebenzylester zugetropft. Dann ließ man unter Rühren langsam auf Raumtemperatur erwärmen und rührte über Nacht bei Raumtemperatur. Das überstehende Lösungsmittel wurde abdekantiert, der Rückstand zweimal mit je 25 ml Tetrahydrofuran verrührt wonach das überstehende Lösungsmittel jeweils abdekantiert wurde. Die vereinigten organischen Phasen wurde eingeengt und das Rohprodukt wurde mittels Kieselgelchromatographie gereinigt (Laufmittel-Gradient: Cyclohexan nach Cyclohexan/Dichlormethan-Gradient 1/1 bis 1/2). Es wurden 2.56 g der Titelverbindung (78 % d. Th.) erhalten.
LC-MS (Methode 2): Rₜ = 0.89 min
MS (ESpos): m/z = 236 (M+H)⁺
¹H-NMR (400 MHz, DMSO-d₆): δ [ppm] = 1.51 (s, 3H), 1.75 - 1.91 (m, 2H), 5.08 (s, 2H), 7.28 - 7.42 (m, 5H), 7.96 (br. s, 1H).

### Beispiel 6A

### ent-Benzyl-[2-cyan(4,4,4-²H₃)butan-2-yl]carbamat (Enantiomer A)

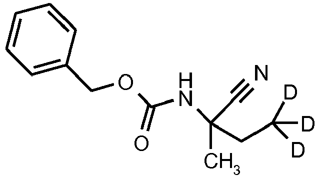

2.56 g *rac*-Benzyl-[2-cyan(4,4,4-²H₃)butan-2-yl]carbamat aus Beispiel 5A wurden durch präparative Trennung an der chiralen Phase in die Enantiomere getrennt [Säule: Daicel Chiralcel OJ-H, 5 µm, 250 x 20 mm, Eluent: 70% iso-Hexan, 30% iso-Propanol, Fluss: 15 ml/min, Temperatur: 47°C, Detektion: 220 nm].
Enantiomer A: 1.03 g (>99% ee)
Rₜ = 7.11 min [Daicel Chiralcel OJ-H, 250 x 4.6 mm, 5 µm, Eluent: 70% iso-Hexan, 30% iso-Propanol, Fluss: 1 ml/min, Temperatur: 50°C, Detektion: 220 nm].

### Beispiel 7A

### ent-Benzyl-[2-cyan(4,4,4-²H₃)butan-2-yl]carbamat (Enantiomer B)

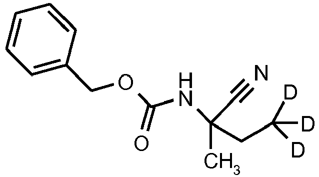

2.56 g *rac*-Benzyl-[2-cyan(4,4,4-²H₃)butan-2-yl]carbamat aus Beispiel 5A wurden durch präparative Trennung an der chiralen Phase in die Enantiomere getrennt [Säule: Daicel Chiralcel OJ-H, 5 µm, 250 x 20 mm, Eluent: 70% iso-Hexan, 30% iso-Propanol, Fluss: 15 ml/min, Temperatur: 47°C, Detektion: 220 nm].
Enantiomer B: 0.99 g (>99% ee)
Rₜ = 8.25 min [Daicel Chiralcel OJ-H, 250 x 4.6 mm, 5 µm, Eluent: 70% iso-Hexan, 30% iso-Propanol, Fluss: 1 ml/min, Temperatur: 50°C, Detektion: 220 nm].

### Beispiel 8A

### ent-Benzyl-[1-amino-2-methyl(4,4,4-²H₃)butan-2-yl]carbamat (Enantiomer A)

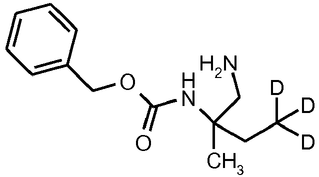

0.50 g (2.13 mmol) *ent*-Benzyl-[2-cyan(4,4,4-²H₃)butan-2-yl]carbamat (Enantiomer A) aus Beispiel 6A wurden in 10 ml 7 N Ammoniak-Lösung in Methanol gelöst und unter Argon mit 0.79 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur abfiltriert, mit Methanol gespült und eingeengt. Es wurden 387 mg (75% d. Th.) der Zielverbindung erhalten, welche ohne weitere Reinigung in die Folgestufe eingesetzt wurde.
LC-MS (Methode 2): Rₜ = 0.50 min
MS (ESpos): m/z = 240 (M+H)⁺

### Beispiel 9A

### ent-Benzyl-[1-amino-2-methyl(4,4,4-²H₃)butan-2-yl]carbamat (Enantiomer B)

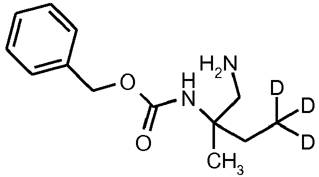

0.50 g (2.13 mmol) *ent*-Benzyl-[2-cyan(4,4,4-²H₃)butan-2-yl]carbamat (Enantiomer B) aus Beispiel 7A wurden in 10 ml 7 N Ammoniak-Lösung in Methanol gelöst und unter Argon mit 0.79 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur abfiltriert, mit Methanol gespült und eingeengt. Es wurden 487 mg (94% d. Th.) der Zielverbindung erhalten, welche ohne weitere Reinigung in die Folgestufe eingesetzt wurde.
LC-MS (Methode 2): Rₜ = 0.53 min
MS (ESpos): m/z = 240 (M+H)⁺

### Beispiel 10A

### rac-2-Amino-2-methyl-4-(trimethylsilyl)butannitril

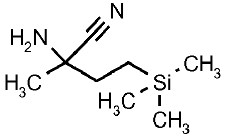

13.0 g (90.10 mmol) 4-(Trimethylsilyl)butan-2-on [käuflich erhältlich oder synthetisierbar nach R. Acerete et al. Journal of Organic Chemistry 2011, 76, 10129-10139] wurden in 25 ml 7 N Ammoniak in Methanol vorgelegt und bei Raumtemperatur mit 5.83 g (118.93 mmol) Natriumcyanid sowie 6.36 g (118.93 mmol) Ammoniumchlorid versetzt und 3 Stunden unter Rückfluss gerührt. Das Reaktionsgemisch wurde abgekühlt, und der enthaltene Feststoff wurde abfiltriert. Das Filtrat wurde unter ohne weitere Reinigung in die Folgestufe eingesetzt wurde.

### Beispiel 11A

### rac-Benzyl-[2-cyan-4-(trimethylsilyl)butan-2-yl]carbamat

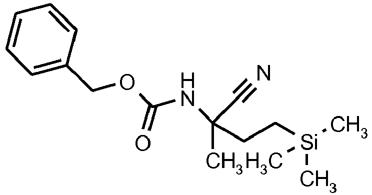

Die Rohlösung von *rac*-2-Amino-2-methyl-4-(trimethylsilyl)butannitril aus Beispiel 10A wurden in 16 ml Wasser vorgelegt und mit 37.36 g (270.35 mmol) Kaliumcarbonat versetzt. Bei 0°C wurden langsam 23.06 g (135.18 mmol) Chlorameisensäurebenzylester zugetropft. Dann ließ man unter Rühren langsam auf Raumtemperatur erwärmen und rührte über Nacht bei Raumtemperatur. Das Reaktionsgemisch wurde abfiltriert und der Rückstand wurde mehrmals mit Tetrahydrofuran gewaschen. Das Filtrat wurde eingeengt und das Rohprodukt wurde mittels Kieselgelchromatographie gereinigt (Laufmittel: Cyclohexan/Essigsäureethylester = 9/1). Es wurden 11.60 g der Titelverbindung (42 % d. Th. über zwei Stufen) erhalten.
LC-MS (Methode 2): Rₜ = 1.23 min
MS (ESpos): m/z = 305 (M+H)⁺
¹H-NMR (400 MHz, DMSO-d₆): δ [ppm] = -0.01 (s, 9H), 0.45 - 0.67 (m, 2H), 1.52 (s, 3H), 1.73 - 1.90 (m, 2H), 2.24 - 2.52 (m, 2H), 5.08 (s, 2H), 7.29 - 7.44 (m, 5H), 7.94 (br. s, 1H).

### Beispiel 12A

### ent-Benzyl-[2-cyan-4-(trimethylsilyl)butan-2-yl]carbamat (Enantiomer A)

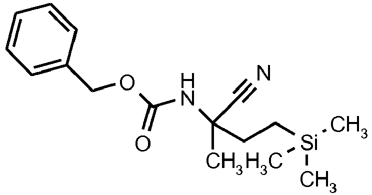

10.0 g *rac*-Benzyl-[2-cyan-4-(trimethylsilyl)butan-2-yl]carbamat aus Beispiel 11A wurden durch präparative Trennung an der chiralen Phase in die Enantiomere getrennt [Säule: Daicel Chiralpak AY-H, 5 µm, 250 x 20 mm, Eluent: 15% Ethanol, 85% iso-Hexan, Fluss: 20 ml/min, Temperatur: 30°C, Detektion: 220 nm].
Enantiomer A: 4.19 g (>99% ee)
Rₜ = 5.24 min [Daicel Chiralpak AY-H, 250 x 4.6 mm, 5 µm, Eluent: 10% Ethanol, 90% iso-Hexan, Fluss: 1 ml/min, Temperatur: 45°C, Detektion: 220 nm].

### Beispiel 13A

### ent-Benzyl-[2-cyan-4-(trimethylsilyl)butan-2-yl]carbamat (Enantiomer B)

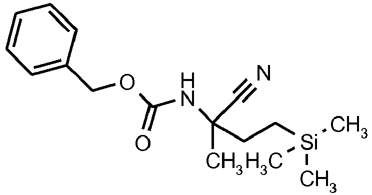

10.0 g *rac*-Benzyl-[2-cyan-4-(trimethylsilyl)butan-2-yl]carbamat aus Beispiel 11A wurden durch präparative Trennung an der chiralen Phase in die Enantiomere getrennt [Säule: Daicel Chiralpak AY-H, 5 µm, 250 x 20 mm, Eluent: 15% Ethanol, 85% iso-Hexan, Fluss: 20 ml/min, Temperatur: 30°C, Detektion: 220 nm].
Enantiomer B: 4.24 g (>99% ee)
Rₜ = 6.89 min [Daicel Chiralpak AY-H, 250 x 4.6 mm, 5 µm, Eluent: 10% Ethanol, 90% iso-Hexan, Fluss: 1 ml/min, Temperatur: 45°C, Detektion: 220 nm].

### Beispiel 14A

### ent-Benzyl-[1-amino-2-methyl-4-(trimethylsilyl)butan-2-yl]carbamat (Enantiomer A)

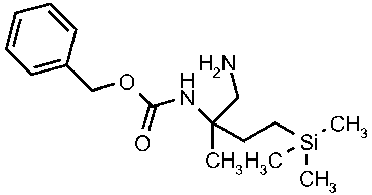

2.0 g (6.57 mmol) *ent*-Benzyl-[2-cyan-4-(trimethylsilyl)butan-2-yl]carbamat (Enantiomer A) aus Beispiel 12A wurden in 31 ml 7 N Ammoniak-Lösung in Methanol gelöst und unter Argon mit 2.44 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur abfiltriert, mit Methanol gespült und eingeengt. Es wurden 1.80 g (87% d. Th.; Reinheit 98%) der Zielverbindung erhalten, welche ohne weitere Reinigung in die Folgestufe eingesetzt wurde.
LC-MS (Methode 16): Rₜ = 1.66 min
MS (ESpos): m/z = 309 (M+H)⁺

### Beispiel 15A

### ent-Benzyl-[1-amino-2-methyl-4-(trimethylsilyl)butan-2-yl]carbamat (Enantiomer B)

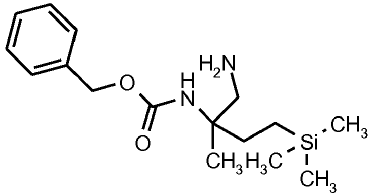

2.0 g (6.57 mmol) *ent*-Benzyl-[2-cyan-4-(trimethylsilyl)butan-2-yl]carbamat (Enantiomer B) aus Beispiel 13A wurden in 31 ml 7 N Ammoniak-Lösung in Methanol gelöst und unter Argon mit 2.44 g Raney-Nickel (50%ige wässrige Aufschlämmung) versetzt. Das Reaktionsgemisch wurde 3 h im Autoklaven bei 20-30 bar hydriert. Die Reaktionsmischung wurde über Kieselgur abfiltriert, mit Methanol gespült und eingeengt. Es wurden 1.72 g (83% d. Th.; Reinheit 98%) der Zielverbindung erhalten, welche ohne weitere Reinigung in die Folgestufe eingesetzt wurde.
LC-MS (Methode 2): Rₜ = 0.78 min
MS (ESpos): m/z = 309 (M+H)⁺

### Beispiel 16A

### ent-Benzyl-{1-[({8-[(2,6-difluorbenzyl)oxy]-2,6-dimethylimidazo[1,2-a]pyrazin-3-yl}carbonyl)amino]-2-methyl(4,4,4-²H₃)butan-2-yl}carbamat Trifluoracetat (Enantiomer A)

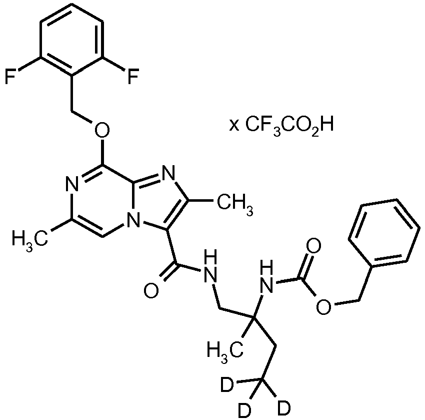

50 mg (0.15 mmol) 8-[(2,6-Difluorbenzyl)oxy]-2,6-dimethylimidazo[1,2-a]pyrazin-3-carbonsäure aus Beispiel 3A wurden mit 63 mg (0.17 mmol) HATU und 0.13 ml (0.75 mmol) N,N-Diisopropylethylamin in 0.5 ml DMF vorgelegt und 10 min bei Raumtemperatur gerührt. Anschließend wurden 44 mg (0.18 mmol; Reinheit 88%) *ent*-Benzyl-[1-amino-2-methyl(4,4,4-²H₃)butan-2-yl]carbamat (Enantiomer A) aus Beispiel 8A zur Reaktionslösung gegeben und 2 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 56 mg der Zielverbindung (53% d. Th.; Reinheit 95%) erhalten.
LC-MS (Methode 2): Rₜ = 1.29 min
MS (ESpos): m/z = 555 (M-TFA+H)⁺

### Beispiel 17A

### ent-Benzyl-{1-[({8-[(2,6-difluorbenzyl)oxy]-2,6-dimethylimidazo[1,2-a]pyrazin-3-yl}carbonyl)amino]-2-methyl-4-(trimethylsilyl)butan-2-yl}carbamat Trifluoracetat (Enantiomer A)

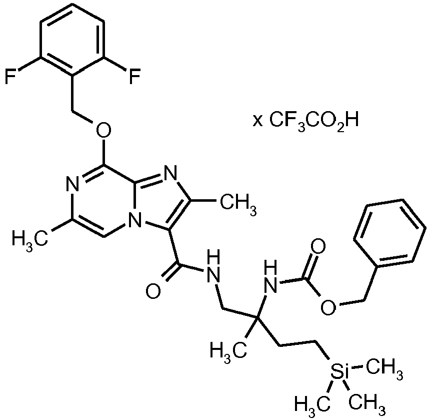

60 mg (0.18 mmol) 8-[(2,6-Difluorbenzyl)oxy]-2,6-dimethylimidazo[1,2-a]pyrazin-3-carbonsäure aus Beispiel 3A wurden mit 75 mg (0.20 mmol) HATU und 0.16 ml (0.90 mmol) N,N-Diisopropylethylamin in 0.6 ml DMF vorgelegt und 10 min bei Raumtemperatur gerührt. Anschließend wurden 91 mg (0.29 mmol) *ent*-Benzyl-[1-amino-2-methyl-4-(trimethylsilyl)butan-2-yl]carbamat (Enantiomer A) aus Beispiel 14A zur Reaktionslösung gegeben und 2 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 97 mg der Zielverbindung (73% d. Th.) erhalten.
LC-MS (Methode 2): Rₜ = 1.51 min
MS (ESpos): m/z = 624 (M-TFA+H)⁺

### Beispiel 18A

### ent-Benzyl-{1-[({8-[(2,6-difluorbenzyl)oxy]-2,6-dimethylimidazo[1,2-a]pyrazin-3-yl}carbonyl)amino]-2-methyl-4-(trimethylsilyl)butan-2-yl}carbamat Trifluoracetat (Enantiomer B)

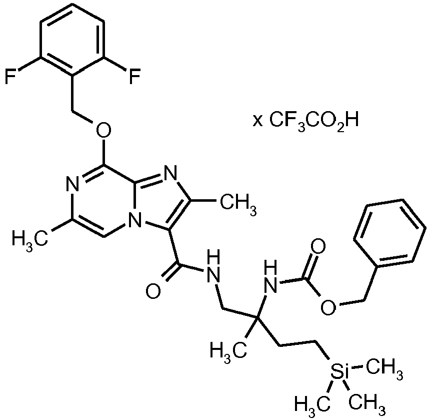

60 mg (0.18 mmol) 8-[(2,6-Difluorbenzyl)oxy]-2,6-dimethylimidazo[1,2-a]pyrazin-3-carbonsäure aus Beispiel 3A wurden mit 75 mg (0.20 mmol) HATU und 0.16 ml (0.90 mmol) N,N-Diisopropylethylamin in 0.6 ml DMF vorgelegt und 10 min bei Raumtemperatur gerührt. Anschließend wurden 91 mg (0.29 mmol) *ent*-Benzyl-[1-amino-2-methyl-4-(trimethylsilyl)butan-2-yl]carbamat (Enantiomer B) aus Beispiel 15A zur Reaktionslösung gegeben und 2 h bei RT gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt, eingeengt und lyophilisiert. Es wurden 94 mg der Zielverbindung (70% d. Th.) erhalten.
LC-MS (Methode 2): Rₜ = 1.51 min
MS (ESpos): m/z = 624 (M-TFA+H)⁺

### Beispiel 19A

### rac-2-Amino-2-[2-(difluormethyl)-2H-tetrazol-5-yl]propan-1-ol

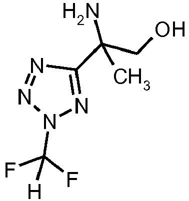

Die Zielverbindung kann durch Entschützung von 1-{[tert-Butyl(dimethyl)silyl]oxy}-2-[2-(difluormethyl)-2H-tetrazol-5-yl]propan-2-amin (herstellbar analog zu Intermediat 300 in WO2014/084312 aus racemischem Ausgangsmaterial) mit Tetrabutylammoniumfluorid (TBAF) in THF bei Raumtemperatur nach literaturbekannten Methoden hergestellt werden.

### Ausführungsbeispiele

### Beispiel 1

### ent-8-[(2,6-Difluorbenzyl)oxy]-N-[(2S)-1-hydroxy-2-(5-methyl-1,3,4-thiadiazol-2-yl)propan-2-yl]-2,6-dimethylimidazo[1,2-a]pyrazin-3-carboxamid

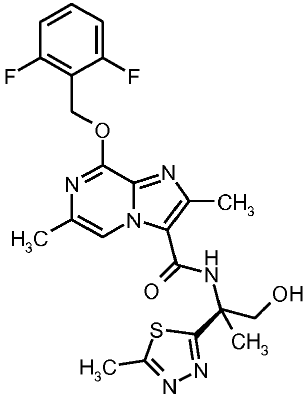

30 mg (0.09 mmol) 8-[(2,6-Difluorbenzyl)oxy]-2,6-dimethylimidazo[1,2-a]pyrazin-3-carbonsäure aus Beispiel 3A wurden mit 37 mg (0.10 mmol) HATU und 123 µl (0.71 mmol) N,N-Diisopropylethylamin in 0.34 ml DMF vorgelegt und 10 min bei Raumtemperatur gerührt. Anschließend wurden 101 mg (0.35 mmol) (2S)-2-Amino-2-(5-methyl-1,3,4-thiadiazol-2-yl)propan-1-ol (herstellbar analog zu Intermediat 307 in WO2014/084312) zur Reaktionslösung gegeben und 2 h bei 60°C gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 28 mg der Zielverbindung (64% d. Th.; Reinheit 98%) erhalten.
LC-MS (Methode 2): Rₜ = 0.94 min
MS (ESpos): m/z = 489 (M+H)⁺
¹H-NMR (400 MHz, DMSO-d₆): δ = 1.81 (s, 3H), 2.35 (s, 3H), 2.59 (s, 3H), 2.69 (s, 3H), 3.82 - 3.97 (m, 2H), 5.45 (t, 1H), 5.56 (s, 2H), 7.18 - 7.26 (m, 2H), 7.52 - 7.62 (m, 1H), 8.27 (s, 1H), 8.31 (s, 1H).

### Beispiel 2

### rac-8-[(2,6-Difluorbenzyl)oxy]-N-{2-[2-(difluormethyl)-2H-tetrazol-5-yl]-1-hydroxypropan-2-yl}-2,6-dimethylimidazo[1,2-a]pyrazin-3-carboxamid

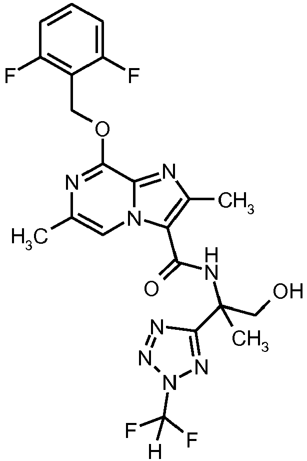

40 mg (0.12 mmol) 8-[(2,6-Difluorbenzyl)oxy]-2,6-dimethylimidazo[1,2-a]pyrazin-3-carbonsäure aus Beispiel 3A wurden mit 47 mg (0.12 mmol) HATU und 102 µl (0.59 mmol) N,N-Diisopropylethylamin in 0.5 ml DMF vorgelegt und 10 min bei Raumtemperatur gerührt. Anschließend wurden 25 mg (0.13 mmol) *rac*-2-Amino-2-[2-(difluormethyl)-2H-tetrazol-5-yl]propan-1-ol Beispiel 19A zur Reaktionslösung gegeben und 2 h bei 60°C gerührt. Dann wurde mit Acetonitril und Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert, eingeengt und lyophilisiert. Es wurden 33 mg der Zielverbindung (55% d. Th.) erhalten.
LC-MS (Methode 2): Rₜ = 1.03 min
MS (ESpos): m/z = 509 (M+H)⁺
¹H-NMR (400 MHz, DMSO-d₆): δ = 1.83 (s, 3H), 2.34 (s, 3H), 2.59 (s, 3H), 3.82 - 3.99 (m, 2H), 5.34 (t, 1H), 5.55 (s, 2H), 7.17 - 7.26 (m, 2H), 7.52 - 7.62 (m, 1H), 8.18 (s, 1H), 8.34 (s, 1H), 8.58 (t, 1H).

### Beispiel 3

### ent-N-[2-Amino-2-methyl(4,4,4-²H₃)butyl]-8-[(2,6-difluorbenzyl)oxy]-2,6-dimethylimidazo[1,2-a]pyrazin-3-carboxamid (Enantiomer A)

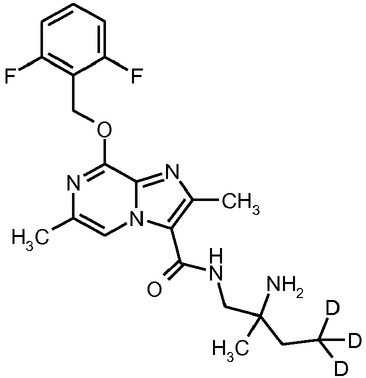

56 mg (0.08 mmol; Reinheit 95%) *ent*-Benzyl-{1-[({8-[(2,6-difluorbenzyl)oxy]-2,6-dimethylimidazo[1,2-a]pyrazin-3-yl}carbonyl)amino]-2-methyl(4,4,4-²H₃)butan-2-yl}carbamat Trifluoracetat (Enantiomer A) aus Beispiel 16A wurden in 2.7 ml Ethanol gelöst und unter Argon mit 30 µl (0.40 mmol) TFA und 6 mg (0.001 mmol) 10%igem Palladium auf Aktivkohle versetzt und 2 Stunden bei Normaldruck hydriert. Die Reaktionslösung wurde mittels Milliporfilter filtriert, mit Ethanol gewaschen und das Filtrat eingeengt. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert und eingeengt. Es wurden 31 mg der Zielverbindung (90% d. Th.) erhalten.
LC-MS (Methode 2): Rₜ = 0.70 min
MS (ESpos): m/z = 421 (M+H)⁺
¹H-NMR (400 MHz, DMSO-d₆): δ = 0.98 (s, 3H), 1.28 - 1.38 (m, 2H), 1.41 (br. s, 2H), 2.34 (s, 3H), 3.15 - 3.28 (m, 2H), 5.55 (s, 2H), 7.18 - 7.25 (m, 2H), 7.52 - 7.62 (m, 1H), 7.82 (br. s, 1H), 8.38 (s, 1H), [weiteres Signal unter Lösungsmittelsignal verborgen].

### Beispiel 4

### ent-N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer A)

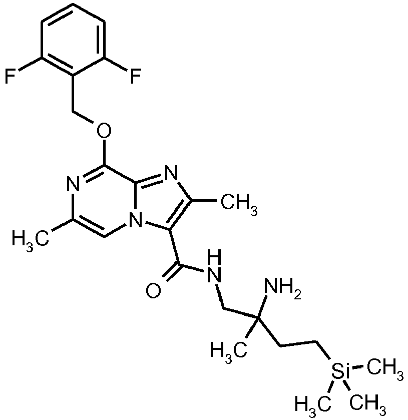

97 mg (0.13 mmol) *ent*-Benzyl-{1-[({8-[(2,6-difluorbenzyl)oxy]-2,6-dimethylimidazo[1,2-a]pyrazin-3-yl}carbonyl)amino]-2-methyl-4-(trimethylsilyl)butan-2-yl}carbamat Trifluoracetat (Enantiomer A) aus Beispiel 17A wurden in 4.5 ml Ethanol gelöst und unter Argon mit 51 µl (0.66 mmol) TFA und 1.5 mg (0.001 mmol) 10%igem Palladium auf Aktivkohle versetzt und 2 Stunden bei Normaldruck hydriert. Die Reaktionslösung wurde mittels Milliporfilter filtriert, mit Ethanol gewaschen und das Filtrat eingeengt. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert und eingeengt. Es wurden 58 mg der Zielverbindung (88% d. Th.) erhalten.
LC-MS (Methode 17): Rₜ = 2.79 min
MS (ESpos): m/z = 490 (M+H)⁺
¹H-NMR (400 MHz, DMSO-d₆): δ = -0.04 (s, 9H), 0.42 - 0.60 (m, 2H), 0.98 (s, 3H), 1.22 - 1.38 (m, 2H), 1.70 (br. s, 2H), 2.34 (s, 3H), 3.17 - 3.28 (m, 2H), 5.54 (s, 2H), 7.17 - 7.25 (m, 2H), 7.52 - 7.62 (m, 1H), 7.83 (br. s, 1H), 8.37 (s, 1H), [weiteres Signal unter Lösungsmittelsignal verborgen].

### Beispiel 5

### ent-N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer B)

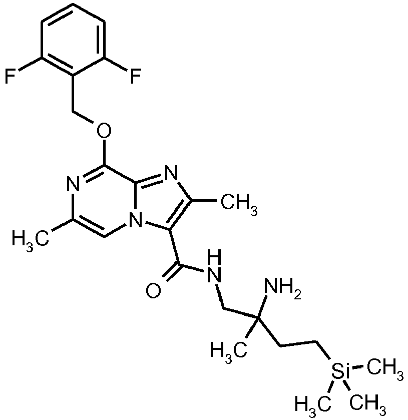

93 mg (0.13 mmol) *ent*-Benzyl-{1-[({8-[(2,6-difluorbenzyl)oxy]-2,6-dimethylimidazo[1,2-a]pyrazin-3-yl}carbonyl)amino]-2-methyl-4-(trimethylsilyl)butan-2-yl}carbamat Trifluoracetat (Enantiomer B) aus Beispiel 18A wurden in 4.3 ml Ethanol gelöst und unter Argon mit 49 µl (0.63 mmol) TFA und 1.4 mg (0.001 mmol) 10%igem Palladium auf Aktivkohle versetzt und 2 Stunden bei Normaldruck hydriert. Die Reaktionslösung wurde mittels Milliporfilter filtriert, mit Ethanol gewaschen und das Filtrat eingeengt. Der Rückstand wurde mit Acetonitril, Wasser und TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und eingeengt. Anschließend wurde der Rückstand in Dichlormethan und wenig Methanol aufgenommen und zweimal mit gesättigter, wässriger Natriumhydrogencarbonatlösung gewaschen. Die wässrige Phase wurde zweimal mit Dichlormethan extrahiert. Die vereinigten organischen Phasen wurden über Natriumsulfat getrocknet, filtriert und eingeengt. Es wurden 52 mg der Zielverbindung (82% d. Th.) erhalten.
LC-MS (Methode 17): Rₜ = 2.80 min
MS (ESpos): m/z = 490 (M+H)⁺
¹H-NMR (400 MHz, DMSO-d₆): δ = -0.04 (s, 9H), 0.42 - 0.60 (m, 2H), 0.98 (s, 3H), 1.22 - 1.38 (m, 2H), 1.54 (br. s, 2H), 2.34 (s, 3H), 3.17 - 3.28 (m, 2H), 5.54 (s, 2H), 7.17 - 7.25 (m, 2H), 7.51 - 7.62 (m, 1H), 7.82 (br. s, 1H), 8.37 (s, 1H), [weiteres Signal unter Lösungsmittelsignal verborgen].

**B. Bewertung der pharmakologischen Wirksamkeit**

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

**Tabelle A:**

| Beispiel | MEC [µM] |
|---|---|
| 1 | 0.65 |
| 2 | 0.10 |
| 3 | 0.65 |
| 4 | 1.0 |
| 5 | 1.0 |

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 Zell-Linie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential - 80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for highresolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007; 56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011; 9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998; 74:230-241.

## Patentansprüche

1. Verbindung der Formel (I) 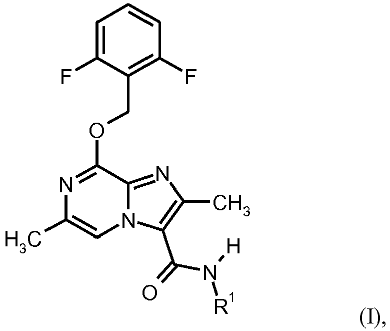 in welcher
R¹ für eine Gruppe der Formel 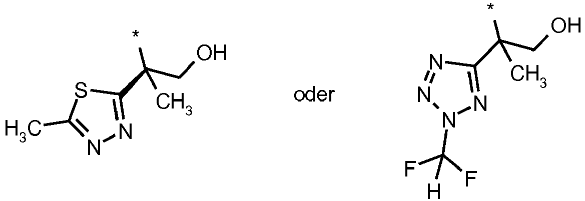 oder 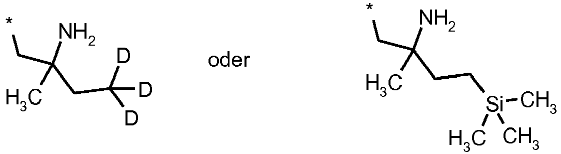 oder 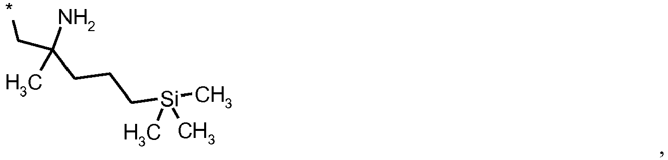 steht, wobei
* für die Anknüpfstelle an das Stickstoffatom steht,
sowie ihre *N*-Oxide, Salze, Solvate, Salze der *N*-Oxide und Solvate der *N*-Oxide und Salze.

2. Verbindung der Formel (I) nach Anspruch 1, in welcher
R¹ für eine Gruppe der Formel 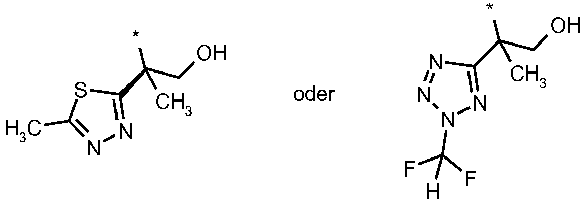 oder 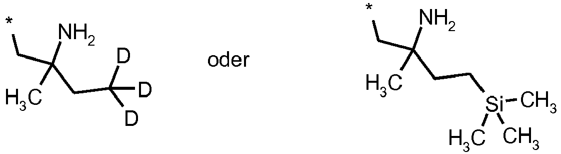 oder, steht, wobei
* für die Anknüpfstelle an das Stickstoffatom steht,
sowie ihre N-Oxide, Salze, Solvate, Salze der N-Oxide und Solvate der N-Oxide und Salze.

3. Verbindung gemäß Anspruch 1 oder 2 mit dem systematischen Namen *ent*-8-[(2,6-Difluorbenzyl)oxy]-N-[(2*S*)-1-hydroxy-2-(5-methyl-1,3,4-thiadiazol-2-yl)propan-2-yl]-2,6-dimethylimidazo[1,2-a]pyrazin-3-carboxamid und der Strukturformel 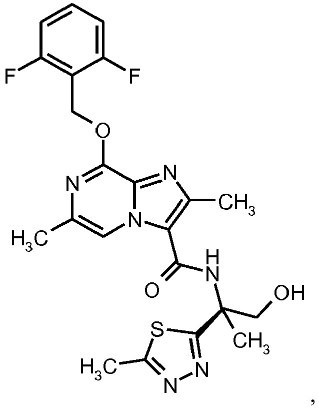 sowie ihre Salze, ihrer Solvate oder der Solvate ihrer Salze.

4. Verbindung gemäß Anspruch 1 oder 2 mit dem systematischen Namen *rac*-8-[(2,6-Difluorbenzyl)oxy]-N-{2-[2-(difluormethyl)-2H-tetrazol-5-yl]-1-hydroxypropan-2-yl}-2,6-dimethylimidazo[1,2-a]pyrazin-3-carboxamid und der Strukturformel 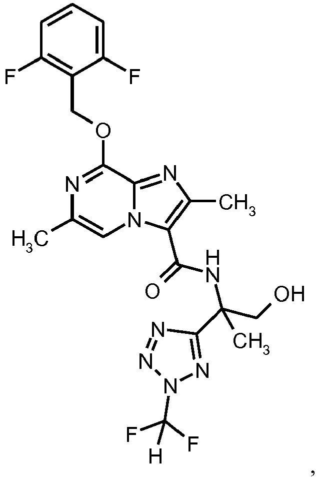 sowie ihre Salze, ihrer Solvate oder der Solvate ihrer Salze.

5. Verbindung gemäß Anspruch 1 oder 2 mit dem systematischen Namen ent-N-[2-Amino-2-methyl(4,4,4-²H₃)butyl]-8-[(2,6-difluorbenzyl)oxy]-2,6-dimethylimidazo[1,2-a]pyrazin-3-carboxamid (Enantiomer A) und der Strukturformel 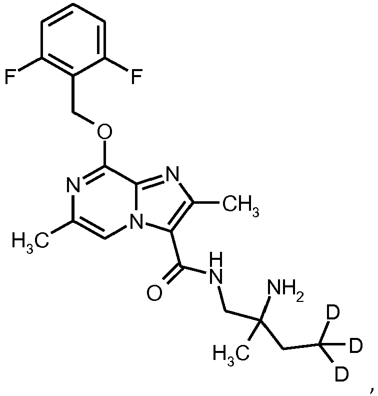 sowie ihre Salze, ihrer Solvate oder der Solvate ihrer Salze.

6. Verbindung gemäß Anspruch 1 oder 2 mit dem systematischen Namen *ent*-N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer A) und der Strukturformel 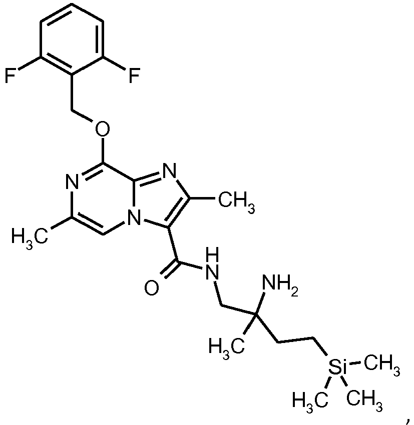 sowie ihre Salze, ihrer Solvate oder der Solvate ihrer Salze.

7. Verbindung gemäß Anspruch 1 oder 2 mit dem systematischen Namen *ent*-N-[2-Amino-2-methyl-4-(trimethylsilyl)butyl]-8-[(2,6-difluorbenzyl)oxy]-2,6-dimethylimidazo[1,2-a]pyrazin-3-carboxamid (Enantiomer B) und der Strukturformel 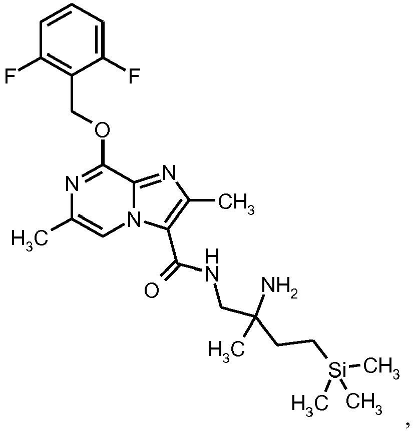 sowie ihre Salze, ihrer Solvate oder der Solvate ihrer Salze.

8. Verfahren zur Herstellung von Verbindungen der Formel (I), wie in den Ansprüchen 1 bis 7 defniert, **dadurch gekennzeichnet, dass** man eine Verbindung der Formel (II) 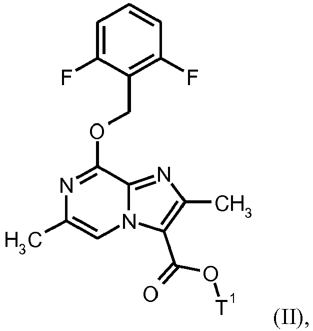 in welcher
T¹ für (C₁-C₄)-Alkyl oder Benzyl steht,
in einem inerten Lösungsmittel in Gegenwart einer geeigneten Base oder Säure zu einer Carbonsäure der Formel (III) 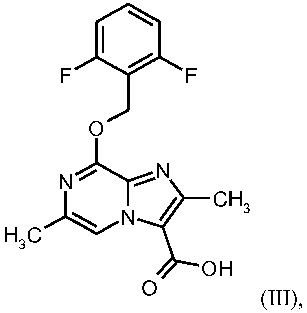 umsetzt und diese in der Folge in einem inerten Lösungsmittel unter Amidkupplungsbedingungen mit einem Amin der Formel (IV-A), (IV-B), (IV-C) oder (IV-D) 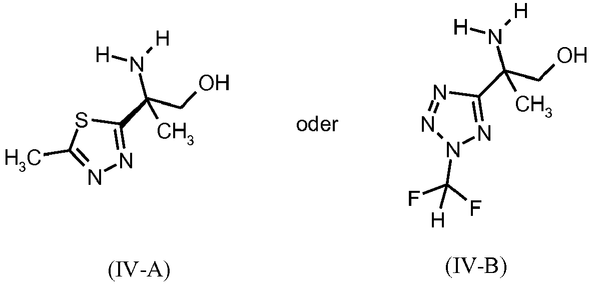 oder 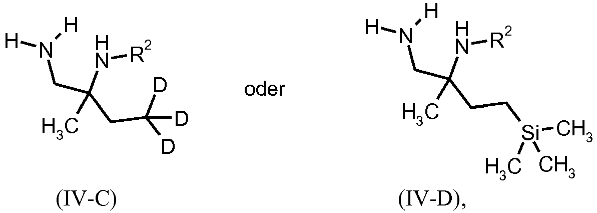 in welchem R² für eine Amino-Schutzgruppe, wie beispielsweise tert.-Butoxycarbonyl, Benzyloxycarbonyl oder Benzyl steht, umsetzt,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

9. Verbindung, wie in einem der Ansprüche 1 bis 7 definiert, zur Behandlung und/oder Prophylaxe von Krankheiten.

10. Verwendung einer Verbindung der Formel (I), wie in den Ansprüchen 1 bis 7 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.

11. Arzneimittel enthaltend eine Verbindung, wie in einem der Ansprüche 1 bis 7 definiert, in Kombination mit einem oder mehreren inerten, nicht-toxischen, pharmazeutisch geeigneten Hilfsstoffen.

12. Arzneimittel enthaltend eine Verbindung, wie in einem der Ansprüche 1 bis 7 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

13. Arzneimittel nach Anspruch 11 oder 12 zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen, Arteriosklerose, Demenzerkrankungen und erektiler Dysfunktion.

## Claims

1. Compound of the formula (I) 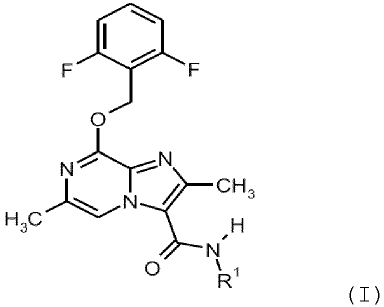 in which
R¹ represents a group of the formula 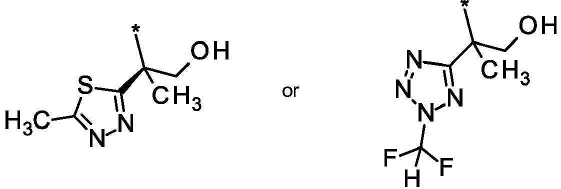 or 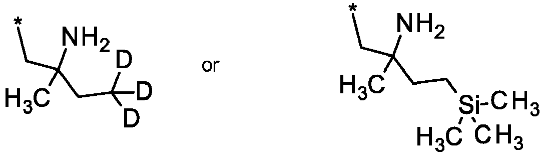 or 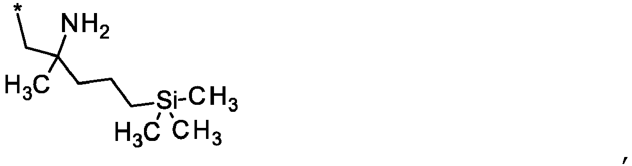 where
* represents the point of attachment to the nitrogen atom,
and the *N*-oxides, salts, solvates, salts of the *N*-oxides and solvates of the *N*-oxides and salts thereof.

2. Compound of the formula (I) according to Claim 1 in which
R¹ represents a group of the formula 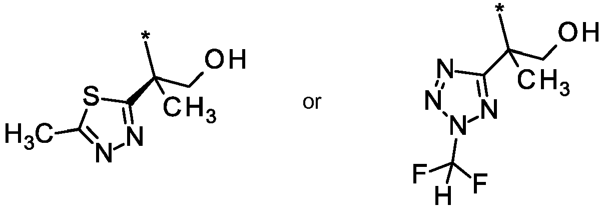 or 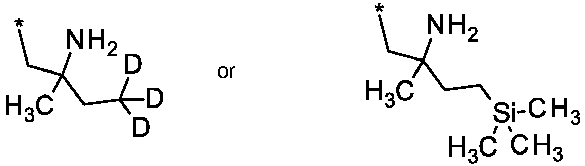 or where
* represents the point of attachment to the nitrogen atom,
and the *N*-oxides, salts, solvates, salts of the *N*-oxides and solvates of the *N*-oxides and salts thereof.

3. Compound according to Claim 1 or 2 having the systematic name *ent*-8-[(2,6-difluorobenzyl)oxy]-N-[(2*S*)-1-hydroxy-2-(5-methyl-1,3,4-thiadiazol-2-yl)propan-2-yl]-2,6-dimethylimidazo[1,2-a]pyrazine-3-carboxamide and the structural formula 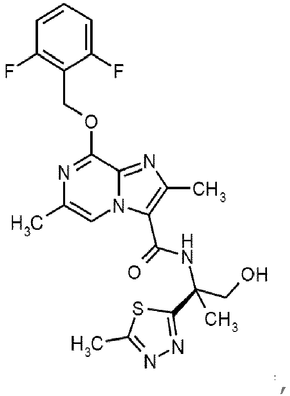 and its salts, its solvates or the solvates of its salts.

4. Compound according to Claim 1 or 2 having the systematic name *rac*-8-[(2,6-difluorobenzyl)oxy]-N-{2-[2-(difluoromethyl)-2H-tetrazol-5-yl]-1-hydroxypropan-2-yl}-2,6-dimethylimidazo[1,2-a]pyrazine-3-carboxamide and the structural formula 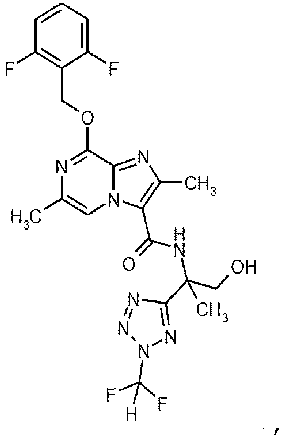 and its salts, its solvates or the solvates of its salts.

5. Compound according to Claim 1 or 2 having the systematic name *ent*-N-[2-amino-2-methyl(4,4,4-²H₃)butyl]-8-[(2,6-difluorobenzyl)oxy]-2,6-dimethylimidozo-[1,2-a]pyrazine-3-carboxamide (enantiomer A) and the structural formula 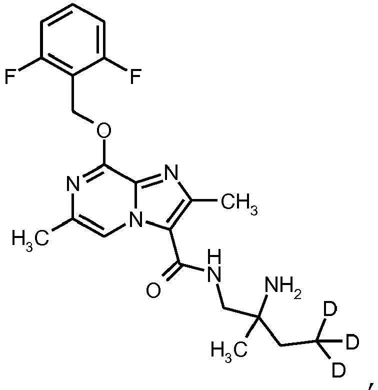 and its salts, its solvates or the solvates of its salts.

6. Compound according to Claim 1 or 2 having the systematic name *ent*-N-[2-amino-2-methyl-4-(trimethylsilyl)butyl]-8-[(2,6-difluorobenzyl)oxy]-2,6-dimethylimidazo[1,2-a]pyrazine-3-carboxamide (enantiomer A) and the structural formula 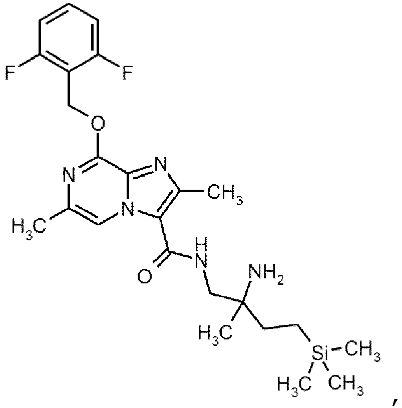 and its salts, its solvates or the solvates of its salts.

7. Compound according to Claim 1 or 2 having the systematic name *ent*-N-[2-amino-2-methyl-4-(trimethylsilyl)butyl]-8-[(2,6-difluorobenzyl)oxy]-2,6-dimethylimidazo[1,2-a]pyrazine-3-carboxamide (enantiomer B) and the structural formula 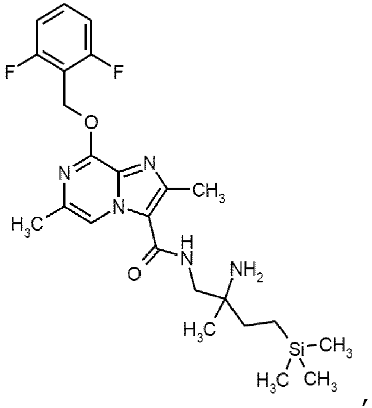 and its salts, its solvates or the solvates of its salts.

8. Process for preparing compounds of the formula (I) as defined in Claims 1 to 7, **characterized in that** a compound of the formula (II) 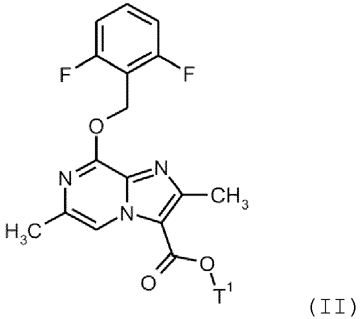 in which
T¹ represents (C₁-C₄)-alkyl or benzyl,
is reacted in an inert solvent in the presence of a suitable base or acid to give a carboxylic acid of the formula (III) 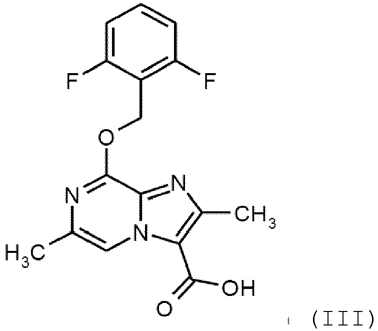 and the latter is subsequently reacted, in an inert solvent under amide coupling conditions, with an amine of the formula (IV-A), (IV-B), (IV-C) or (IV-D) 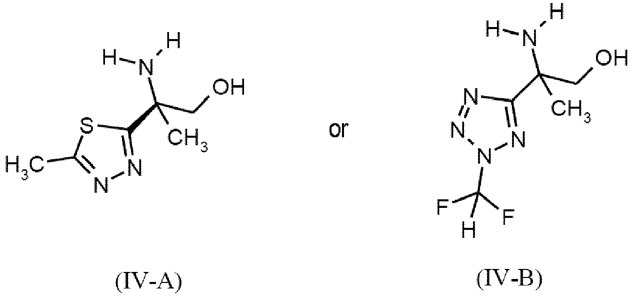 or 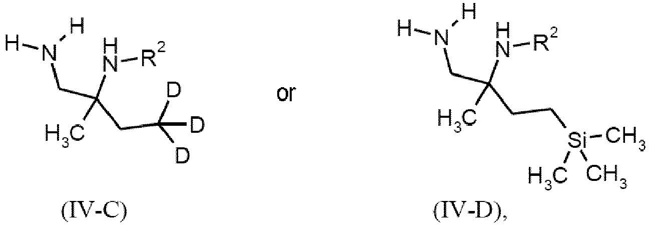 in which R² represents an amino protecting group such as, for example, tert-butoxycarbonyl, benzyloxycarbonyl or benzyl,
then any protective groups present are detached, and the resulting compounds of the formula (I) are optionally converted with the appropriate (i) solvents and/or (ii) acids or bases to the solvates, salts and/or solvates of the salts thereof.

9. Compound as defined in any of Claims 1 to 7 for treatment and/or prophylaxis of diseases.

10. Use of a compound of the formula (I) as defined in Claims 1 to 7 for producing a medicament for the treatment and/or prophylaxis of heart failure, angina pectoris, hypertension, pulmonary hypertension, ischaemias, vascular disorders, renal insufficiency, thromboembolic disorders, fibrotic disorders, arteriosclerosis, dementia disorders and erectile dysfunction.

11. Medicament comprising a compound as defined in any of Claims 1 to 7 in combination with one or more inert, nontoxic, pharmaceutically suitable excipients.

12. Medicament comprising a compound as defined in any of Claims 1 to 7 in combination with a further active compound selected from the group consisting of organic nitrates, NO donors, cGMP-PDE inhibitors, antithrombotic agents, hypotensive agents and lipid metabolism modifiers.

13. Medicament according to Claim 11 or 12 for use in the treatment and/or prophylaxis of heart failure, angina pectoris, hypertension, pulmonary hypertension, ischemias, vascular disorders, renal insufficiency, thromboembolic disorders, fibrotic disorders, arteriosclerosis, dementia disorders and erectile dysfunction.

## Revendications

1. Composé de formule (I) 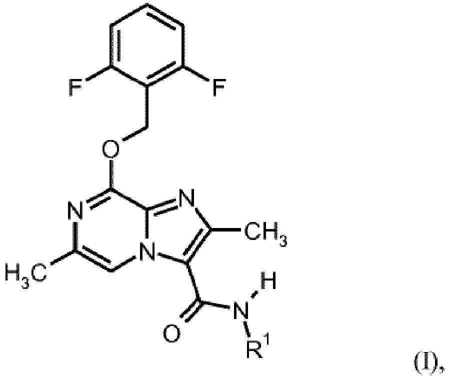 dans laquelle
R¹ représente un groupe de formule 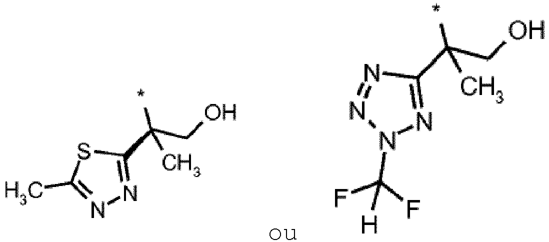 ou 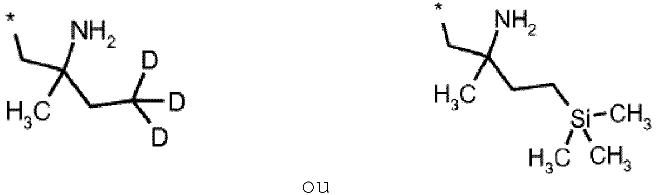 ou 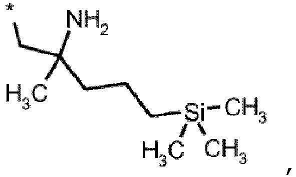
* représentant l'emplacement de liaison à l'atome d'azote,
ainsi que ses N-oxydes, sels, solvates, sels des N-oxydes et solvates des N-oxydes et des sels.

2. Composé de formule (I) selon la revendication 1, dans lequel
R¹ représente un groupe de formule 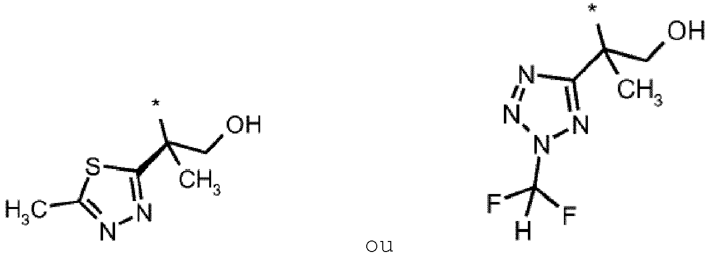 ou 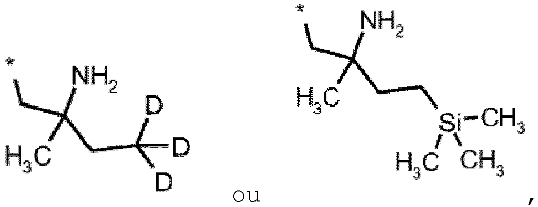
* représentant l'emplacement de liaison à l'atome d'azote,
ainsi que ses N-oxydes, sels, solvates, sels des N-oxydes et solvates des N-oxydes et des sels.

3. Composé selon la revendication 1 ou 2, ayant le nom systématique *ent*-8-[(2,6-difluorobenzyl)oxy]-N-[(2S)-1-hydroxy-2-(5-méthyl-1,3,4-thiadiazol-2-yl)propan-2-yl]-2,6-diméthylimidazo[1,2-a]pyrazine-3-carboxamide et la formule structurale 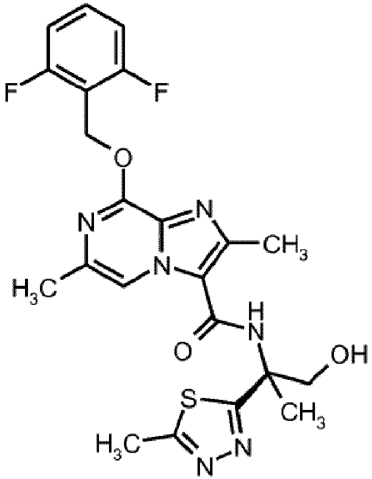 ainsi que ses sels, ses solvates ou les solvates de ses sels.

4. Composé selon la revendication 1 ou 2, ayant le nom systématique *rac*-8-[(2,6-difluorobenzyl)oxy]-N-{2-[2-(difluorométhyl)-2H-tétrazol-5-yl]-1-hydroxypropan-2-yl}-2,6-diméthylimidazo[1,2-a]pyrazine-3-carboxamide et la formule structurale 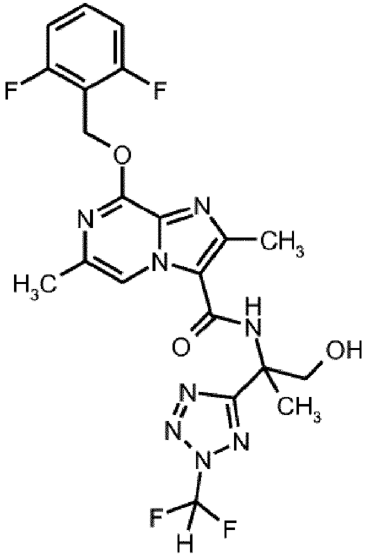 ainsi que ses sels, ses solvates ou les solvates de ses sels.

5. Composé selon la revendication 1 ou 2, ayant le nom systématique *ent*-N-[2-amino-2-méthyl(4,4,4-²H₃)butyl]-8-[(2,6-difluorobenzyl)oxy]-2,6-diméthylimidazo[1,2-a]pyrazine-3-carboxyamide (énantiomère A) et la formule structurale 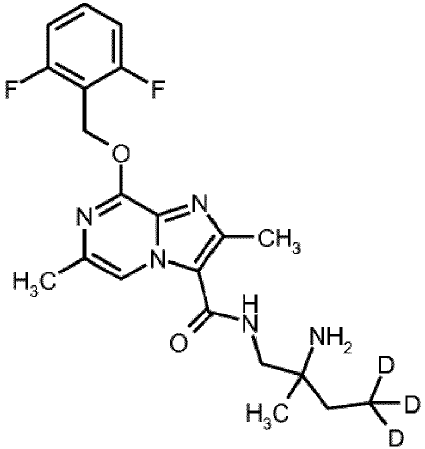 ainsi que ses sels, ses solvates ou les solvates de ses sels.

6. Composé selon la revendication 1 ou 2, ayant le nom systématique *ent*-N-[2-amino-2-méthyl-4-(triméthylsilyl)butyl]-8-[(2,6-difluorobenzyl)oxy]-2,6-diméthylimidazo[1,2-a]pyrazine-3-carboxyamide (énantiomère A) et la formule structurale 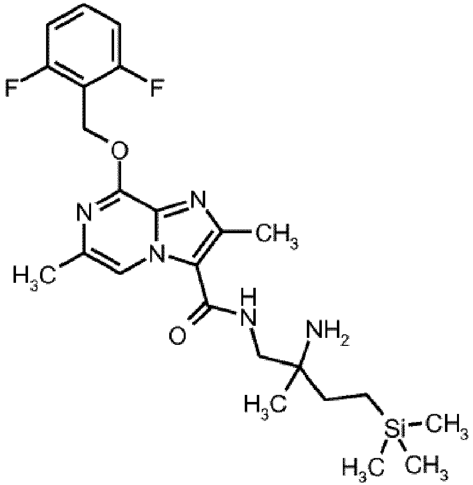 ainsi que ses sels, ses solvates ou les solvates de ses sels.

7. Composé selon la revendication 1 ou 2, ayant le nom systématique *ent*-N-[2-amino-2-méthyl-4-(triméthylsilyl)butyl]-8-[(2,6-difluorobenzyl)oxy]-2,6-diméthylimidazo[1,2-a]pyrazine-3-carboxyamide (énantiomère B) et la formule structurale 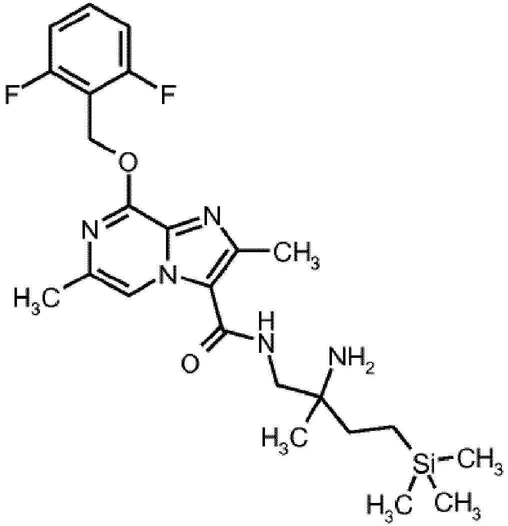 ainsi que ses sels, ses solvates ou les solvates de ses sels.

8. Procédé de fabrication de composés de formule (I), tels que définis dans les revendications 1 à 7, **caractérisé en ce qu'**un composé de formule (II) 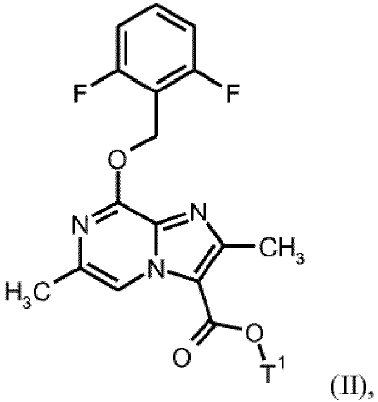 dans laquelle
T¹ représente alkyle en (C₁-C₄) ou benzyle,
est mis en réaction dans un solvant inerte en présence d'une base ou d'un acide approprié pour former un acide carboxylique de formule (III) 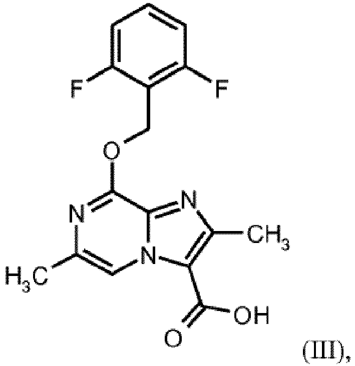 puis celui-ci est mis en réaction dans un solvant inerte en conditions de couplage d'amide avec une amine de formule (IV-A), (IV-B), (IV-C) ou (IV-D) 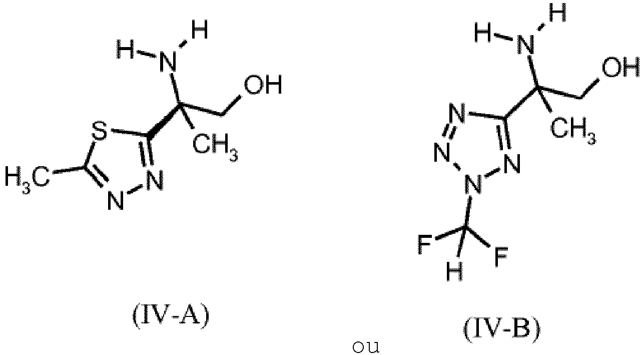 ou 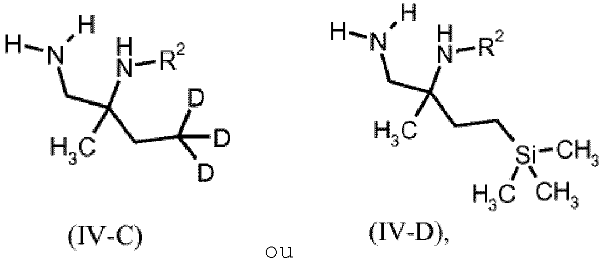 dans lesquelles R² représente un groupe protecteur amino, tel que par exemple tert.-butoxycarbonyle, benzyloxycarbonyle ou benzyle,
puis les groupes protecteurs éventuellement présents sont clivés, et les composés de formule (I) résultants sont éventuellement transformés avec (i) les solvants et/ou (ii) les acides ou les bases appropriés en leurs solvates, sels et/ou solvates des sels.

9. Composé, tel que défini dans l'une quelconque des revendications 1 à 7, pour le traitement et/ou la prophylaxie de maladies.

10. Utilisation d'un composé de formule (I), tel que défini dans les revendications 1 à 7, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques, l'artériosclérose, les maladies de démence et le dysfonctionnement érectile.

11. Médicament contenant un composé tel que défini dans l'une quelconque des revendications 1 à 7, en combinaison avec un ou plusieurs adjuvants inertes, non toxiques, pharmaceutiquement appropriés.

12. Médicament contenant un composé tel que défini dans l'une quelconque des revendications 1 à 7, en combinaison avec un agent actif supplémentaire, choisi dans le groupe constitué par les nitrates organiques, les donneurs de NO, les inhibiteurs de cGMP-PDE, les agents à activité antithrombotique, les agents abaissant la tension artérielle et les agents modifiant le métabolisme lipidique.

13. Médicament selon la revendication 11 ou 12, destiné à une utilisation dans le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques, l'artériosclérose, les maladies de démence et le dysfonctionnement érectile.
